# EUROPEAN PATENT SPECIFICATION

(11) **EP 2 441 490 B1**
(45) Date of publication and mention of the grant of the patent: **22.05.2013**
(21) Application number: 11183045.1
(22) Date of filing: 28.09.2011
(51) Int. Cl.: A61M 39/02

(54) **Subcutaneous implantable port and method for producing the same**
Subkutan implantierbarer Port und Herstellungsverfahren dafür
Port sous-cutané implantable et son procédé de production

(30) Priority: 29.09.2010 JP 2010218888
(43) Date of publication of application: 18.04.2012
(73) Proprietor: Covidien LP, Mansfield, MA 02048 (US)
(72) Inventor: Mikami, Hidetomo, Shizuoka 437-0004 (JP)
(74) Representative: Gray, James

(56) References cited:
- EP-A2- 1 832 252
- WO-A1-00/33901
- WO-A2-02/074381
- DE-U1-202005 013 295

## Description

### BACKGROUND

### Technical Field

The present disclosure relates to a subcutaneous implantable port configured for implantation under a patient's skin, and to a method for producing the same.

### Description of Related Art

Conventional subcutaneous implantable ports are known in the medical art and may be configured for administering anticancer drugs, nutrients or the like to a patient over prolonged periods of time. Typically, subcutaneous implantable ports are used while connected to a catheter that is placed in a blood vessel and while implanted under the skin of a patient's chest region or the like. Conventional subcutaneous implantable ports have a structure in which a septum including an elastic member is disposed inside a housing made of synthetic resin. The housing consists of a cup-shaped base having a bottom and a cover for covering the base. Inside the housing, an injection liquid chamber is defined below the septum. An injection needle is then inserted into the septum through the skin, and the tip end of the needle is inserted into the injection liquid chamber. Drugs are injected into the blood vessel through the catheter by introducing drugs in this state.

A conventional subcutaneous implantable port has been proposed in which a metal bottom plate is provided at the bottom part of the housing in order to prevent the injection needle from penetrating the bottom part of the housing. A bottom plate such as this is placed at the bottom part of the base and fixed by heat-sealing, for example.

Furthermore, in other conventional technology, a retaining lip is formed beforehand as a single piece with the base. The bottom plate is then disposed so as to cause the retaining lip to deform, whereby the bottom plate is fixed (see Japanese Unexamined Patent Application Publication 2007-643.

However, the following problems are encountered with the conventional technology mentioned above.

For example, in the instance where the bottom plate is fixed by heat-sealing, the base and the cover are sometimes fixed by means of ultrasonic welding as a process subsequent to the heat-sealing. However, the ultrasonic waves in this process cause oscillation of the bottom plate which is not intended to be oscillated from the outset. As a result, the heat-sealed area around the bottom plate melts, which may result in the bottom plate shifting position or becoming detached, etc.

Furthermore, the conventional technology in which the bottom plate is fixed by means of the retaining lip involves a complex process because it is necessary to deform the retaining lip. Moreover, the fixing of the bottom plate is also likely to become inadequate. Alternative methods have also been considered, including a method in which the bottom plate is implanted in the base by means of insert-molding, and a method in which the bottom plate is fixed to the base using separate fixing parts. However, such processes are inevitably complex, and unfortunately lead to increased production costs.

EP1832252 teaches an external pressure-based gastric band adjustment system and method.

DE202005013295 discloses an implant for continuous application of medicine comprising filter and collection compartment, which implant comprises the combination of features of the preamble of claim 1.

WO02/074381 teaches an implantable refillable and ported controlled release drug delivery device.

WO00/33901 discloses an implantable vascular access device comprising a port base with a metallic dish insert molded into the bottom of the reservoir.

The present disclosure has been devised in view of the issues outlined above, and the object thereof lies in providing a subcutaneous implantable port in which a bottom plate for preventing injection needle penetration can be securely fixed, without increased costs. Another object of the present disclosure lies in providing a method which makes it possible to produce the abovementioned superior subcutaneous implantable port in a relatively simple manner.

### SUMMARY

The present disclosure provides a subcutaneous implantable port including: a housing having a structure in which a cup-shaped base having a bottom which includes a recess that defines an injection liquid chamber is covered by a cover. A septum which can be penetrated by an injection needle is disposed inside the housing in a state in which it is partly exposed through a hole in the cover. A bottom plate which is made of a harder material than the base and is disposed on the bottom part inner surface of the base. A catheter connection part is disposed on the outside surface of the housing. The subcutaneous implantable port being characterized in that the recess has an opening of enlarged diameter; the bottom plate is a member having a plurality of corner parts on the outer peripheral edge thereof and the plurality of corner parts bite into the inside surface of the recess as the bottom plate is press-fitted into the recess along the recess depth direction, whereby the bottom plate is fixed to the bottom part of the base.

In an embodiment, the plurality of corner parts of the bottom plate are made to bite into the inside surface of the recess by means of press-fitting to securely fix the bottom plate to the bottom part of the base without having to rely on a retaining lip or separate fixing parts.

In an embodiment, the bottom plate is polygonal when viewed as a plane, and the recess is circular when viewed as a plane, and the maximum dimension of the bottom plate in the planar direction is equal to or less than the inner diameter of the opening in the recess and greater than the inner diameter of the deepest part of the recess.

In accordance with the instant disclosure, the bottom plate can be easily positioned into the opening in the recess and attached by press-fitting. Furthermore, the plurality of corner parts of the attached bottom plate can be made to bite into the inside surface of the recess as the bottom plate enters the deepest part of the recess. It should be noted that the abovementioned polygonal shape is preferably a regular polygonal shape, and this has the advantage that the amount by which the corner parts each bite in is equal.

In an embodiment, a plurality of resin fusion parts are present in correspondence to the places where the plurality of corner parts bite into the inside surface of the recess, and the plurality of resin fusion parts cover each of the plurality of corner parts.

For example, where the bottom plate has a circular shape without corner parts, a relatively thin resin fusion part may be formed around the whole circumference of the bottom plate. In certain embodiments, the resin fusion parts are not present around the whole circumference of the bottom plate, rather the plurality of resin fusion parts are present in parts, and therefore the resin fusion parts are unlikely to become thin, providing improved fixing strength for the bottom plate as a result of the plurality of corner parts each being covered by the plurality of resin fusion parts.

In embodiments, the thickness of the outer peripheral edges of the bottom plate where the plurality of corner parts are present is less than the thickness of the central part of the bottom plate.

In accordance with the instant disclosure, the resistance to receiving the plurality of corner parts is reduced when the plurality of corner parts are made to bite into the inside surface of the recess. Consequently, the plurality of corner parts can easily and reliably be made to bite into the inside surface of the recess; this means that it is possible to increase the amount by which each corner part bites in, so the bottom plate can be fixed more securely.

The present disclosure also provides a method for producing the subcutaneous implantable port. The method includes a bottom plate fixing step in which the bottom plate is press-fitted into the recess along the recess depth direction under unheated conditions, and the plurality of corner parts are made to bite into the inside surface of the recess so that the bottom plate is fixed to the bottom part of the base; and an ultrasonic welding step in which, after the bottom plate fixing step, ultrasonic waves are applied in order to fuse the base and the cover.

In accordance with the present disclosure, when the bottom plate is press-fitted into the recess along the recess depth direction in the bottom plate fixing step, the plurality of corner parts bite into the inside surface of the recess, and the bottom plate is fixed to the bottom part of the base. When ultrasonic waves are applied in the subsequent ultrasonic welding step, the base and cover are welded into a single piece, thereby forming the housing. Therefore, according to this production method, there is no particular increase in the number of steps or components when the bottom plate is fixed, and therefore it is possible to produce the abovementioned superior subcutaneous implantable port in a relatively simple manner.

In an embodiment, in the ultrasonic welding step, ultrasonic waves are also applied to the places where the plurality of corner parts bite into the inside surface of the recess in order to form a plurality of resin fusion parts, whereby the bottom plate is simultaneously fixed by fusion to the base.

In accordance with the present disclosure, not only is the boundary region of the base and the cover fused and welded when ultrasonic waves are applied in the ultrasonic welding step, the biting portions of the bottom plate are also oscillated to produce heat. As a result thereof, the material around the biting portions is fused, and resin fusion parts which cover each of the corner parts of the bottom plate are formed. As a result, the bottom plate is more securely fixed by welding it to the base and in less steps when compared to conventional fixation methods.

In accordance with the instant disclosure, it is possible to provide a subcutaneous implantable port in which the bottom plate for preventing injection needle penetration can be securely fixed, without increased costs. Moreover, it is possible to provide a method which makes it possible to produce the abovementioned superior subcutaneous implantable port in a relatively simple manner.

### BRIEF DESCRIPTION OF THE DRAWINGS

Various embodiments of the present disclosure are described hereinbelow with references to the drawings, wherein:
Fig. 1(a) is a front view of the subcutaneous implantable port according to an embodiment of the present disclosure;
Fig. 1(b) is a plan view of the subcutaneous implantable port depicted in Fig. 1(a);
Fig. 2 is a cross-sectional view taken along section line A-A in Fig. 1(b);
Fig. 3(a) is a plan view of the bottom plate of the subcutaneous implantable port depicted in Fig. 2;
Fig. 3(b) is a cross-sectional view taken along section line B-B of Fig. 3(a) illustrating the bottom plate of Fig. 3(a);
Fig. 3(c) is a plan view of the base before the bottom plate has been fixed thereto;
Fig. 4 is a cross-sectional view illustrating the bottom plate positioned on a bottom portion of the base in a pre-affixed configuration;
Fig. 5 is a plan view showing the bottom plate affixed on the bottom portion of the base;
Fig. 6 (a) is an enlarged cross-sectional view of the main parts (the region P1 in Fig. 2) showing a state prior to an ultrasonic welding step according to an embodiment of the instant disclosure;
Fig. 6(b) is an enlarged cross-sectional view of the main parts showing the state thereof following ultrasonic welding;
Fig. 6(c) is an enlarged cross-sectional view of the main parts showing a state thereof following ultrasonic welding in an example of the prior art; and
Figs. 7(a)-7(f) are plan views of bottom plates according to various embodiments of the present disclosure.

### DETAILED DESCRIPTION

Detailed embodiments of the present disclosure are disclosed herein; however, the disclosed embodiments are merely examples of the disclosure, which may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure.

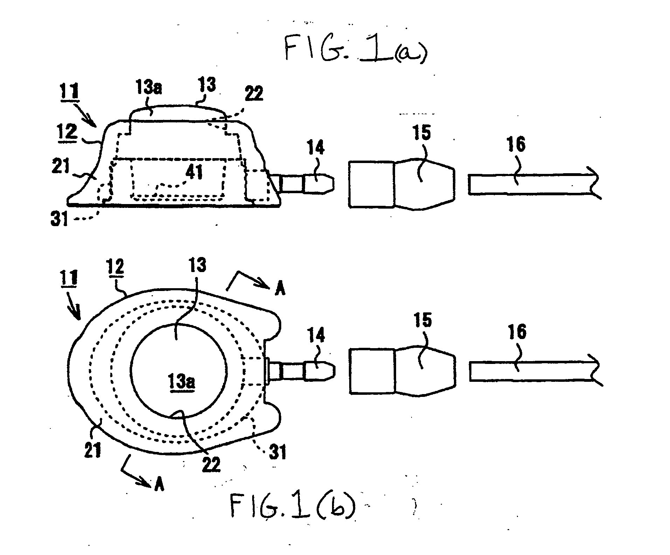

The subcutaneous implantable port of a specific embodiment of the present disclosure will be hereinafter described in detail with reference to Figs. 1 to 7.

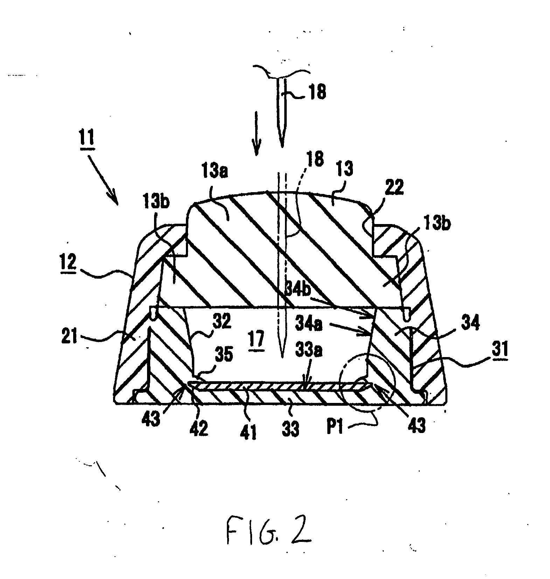

A subcutaneous implantable port 11 according to an embodiment of the present disclosure is used for administering anticancer drugs or nutrients to a patient over a prolonged period of time. A housing 12 which forms part of the subcutaneous implantable port 11 is formed by a base 31 and a cover 21. The base 31 is a molded article made of a general-purpose synthetic resin material such as polypropylene. As shown in Fig. 2, the base 31 comprises a bottom part 33 and a side wall 34 which extends upwards from the outer peripheral part of the bottom part 33. The top end of the side wall 34 is open. A flange is formed on the outer peripheral part at the bottom end of the side wall 34. A cylindrical protrusion 36 which incorporates a catheter connection part 14 is provided on part of the flange. The base 31 has a recess 32 that defines an injection liquid chamber 17 within the side wall 34, and the overall shape is that of a cup with a bottom.

Cover 21 is formed to be slightly bigger than the base 31, and a circular hole 22 is formed in the center of the upper surface of the cover 21. The cover 21 is also a molded article made of a general-purpose synthetic resin material such as polypropylene, similar to that of the base 31. The cover 21 is fitted to the base 31 in such a way as to cover the base 31 from the top part thereof, and the two components are fixed together by means of ultrasonic welding. It should be noted that in this mode of embodiment, the cover 21 and the base 31 are fixed together by means of ultrasonic welding, but they may be fixed by adhesion or by interlocking, etc.

As shown in Fig. 2, a septum 13 may be made from an elastic body (preferably a silicone resin) such as, for example, a hard rubber, and is disposed inside the housing 12. The septum 13 is a circular member when viewed from above, and the central part 13a thereof is formed to be thicker so as to protrude at the upper surface. The outer peripheral part 13b of the septum 13 is fixed by pinching between the top end of the side wall 34 of the base 31 and the outer peripheral part on the rear surface side at the top end of the cover 21. The thick central part 13a of the septum 13 projects outwards through the hole 22 in the cover 21, and can be penetrated by an injection needle 18. It should be noted that the septum 13 seals the injection liquid chamber 17 in such a way that drugs do not leak from the injection chamber 17.

A prescribed region on the outer peripheral part of the cover 21 is formed with a recess, and the catheter connection part 14 is disposed in this region. The catheter connection part 14 passes through the side wall 34 of the base 31 while also providing communication between the region outside the base 31 and the recess 32. A catheter fixing implement 15 which is made of synthetic resin is provided at the connection end of a catheter 16. When the catheter 16 has been inserted into the catheter connection part 14, the catheter fixing implement 15 is engaged or screwed on in order to attach it to the catheter connection part 14. This attachment allows the catheter 16 to be securely fixed to the catheter connection part 14.

The structure for fixing the bottom plate in the subcutaneous implantable port 11 according to an embodiment of the present disclosure will be described next.

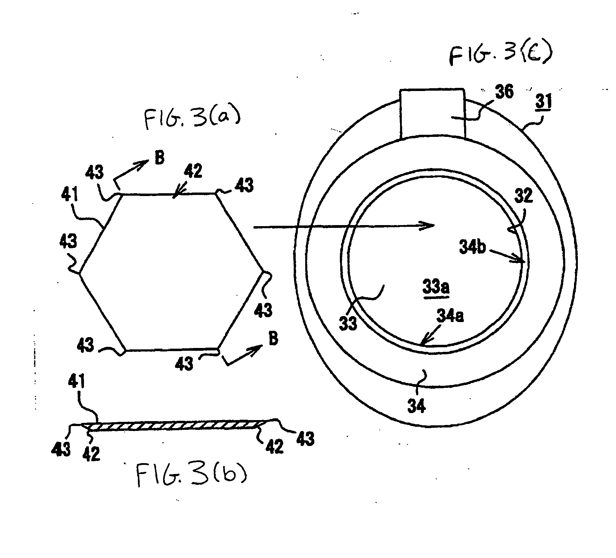

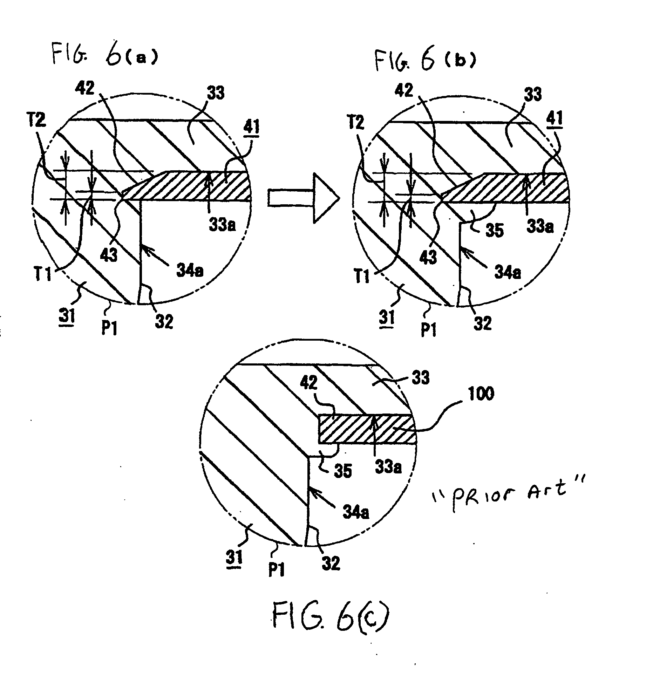

As shown in Figs. 3(a) and 3(b), the subcutaneous implantable port 11 includes a bottom plate 41. The bottom plate 41 according to this mode of embodiment is a member made of a metal which is harder than the base 31, cover 21 and septum 13 (specifically titanium or titanium alloy). The bottom plate 41 has the shape of a regular hexagon when viewed from above, with six corner parts 43 at the peripheral edge 42 thereof. The outer peripheral edge 42 of the bottom plate 41 where the corner parts 43 are present is formed in a tapered manner by bevelling the lower surface side of the bottom plate 41. The thickness T1 of the outer peripheral edge 42 of the bottom plate 41 where the corner parts 43 are present is therefore somewhat less than the thickness T2 at the center of the bottom plate 41 (see Figs. 3(b) and 6(a) and 6(b)).

Furthermore, as shown in Fig. 3(c), the recess 32 in the base 31 is formed with a different shape to that of the bottom plate 41 when viewed from above (in this instance it is circular). It should be noted that the recess 32 according to this mode of embodiment includes an opening 34b of enlarged diameter to take account of the ease of attachment of the bottom plate 41 (see Fig. 2). To be more specific, the region at the top half of the inside surface 34a of the recess 32 is slightly inclined with respect to the bottom part inner surface 33a, forming a tapered surface which tapers towards the inside of the recess 32. The region at the bottom half of the inside surface 34a of the recess 32, on the other hand, is perpendicular with respect to the bottom part inner surface 33a and has no tapered surface in particular

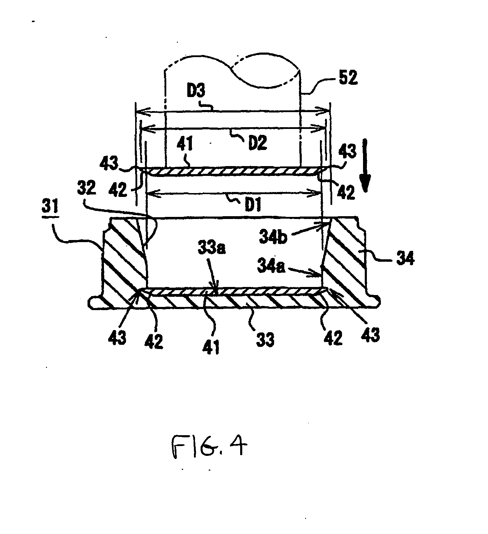

In this embodiment, the respective dimensions of the bottom plate 41 and the recess 32 are set as follows. Here, the maximum dimension of the bottom plate 41 in the planar direction is "D2". Furthermore, the inner diameter at the deepest part of the recess 32 is "D1" and the inner diameter of the opening 34b is "D3". In this instance, the relationship is set in such a way that D1 < D2 ≤ D3 (see Fig. 4). Having the maximum dimension D2 of the bottom plate 41 in the planar direction equal to or less than the inner diameter D3 of the opening 34b in the recess 32 allows the bottom plate 41 to be easily positioned into the opening 34b in the recess 41 and attached by press-fitting. Furthermore, having the maximum dimension D2 of the bottom plate 41 in the planar direction greater than the inner diameter D1 of the deepest part of the recess 32 allows the plurality of corner parts 42 of the attached bottom plate 41 to bite into the inside surface 34a of the recess 32 as the bottom plate 41 enters the deepest part of the recess 32.

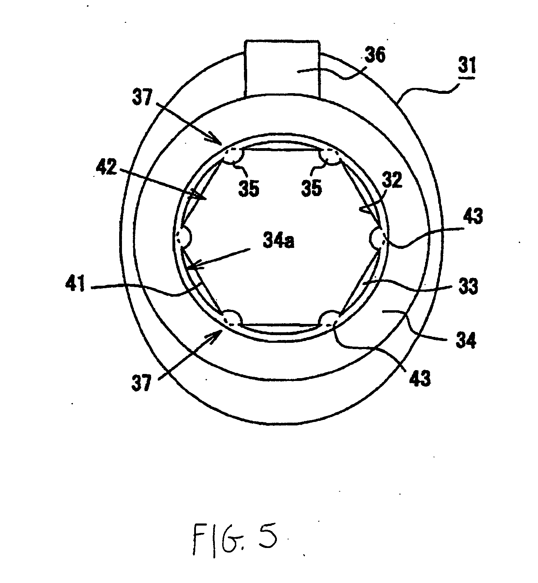

The bottom plate 41 having the above structure is disposed in a state of contact with the bottom part inner surface 33a of the base 31. Furthermore, the six corner parts 43 of the bottom plate 41 each bite into the inside surface 34a of the recess 32 when the bottom plate 41 is press-fitted into the recess 32 along the recess depth direction (see Figs. 4 and 5). As a result, the bottom plate 41 is fixed to the bottom part 33 of the base 31

Furthermore, as shown in Figs. 2, 5 and 6(b), a plurality of resin fusion parts 35 are present in correspondence to the places where the plurality of corner parts 42 bite into the inside surface 34a of the recess 32. The plurality of resin fusion parts 35 cover each of the plurality of corner parts 42.

The method for producing the subcutaneous implantable port 11 according to an embodiment of the present disclosure will be described next.

First, the base 31, cover 21, septum 13 and bottom plate 41 etc. are prepared. The bottom plate 41 is then held by suction-attachment using a specific tool 52 (e.g., a vacuum chuck etc.), and is placed with the bevelled surface of the bottom plate 41 facing the opening in the recess 32 (see Fig. 4).

The bottom plate 41 is then press-fitted into the recess 32 along the recess depth direction using the tool 52, and the bottom plate 41 is moved to a position in which it comes into contact with the bottom part inner surface 33a of the base 31. During the press-fitting operation, no particular heating is used, and the operation is carried out at normal temperature. As a result of this unheated press-fitting, the plurality of corner parts 43 are made to bite into the inside surface 34a of the recess 32. The bottom plate 41 is supported at six points around the outer peripheral edge, and the bottom plate 41 is securely fixed to the bottom part 33 (see Fig. 4).

After the bottom plate fixing step, the septum 13 is positioned so as to be held between the base 31 and the cover 21, and the ultrasonic welding step is carried out. When this step is carried out, ultrasonic waves are applied to the boundary region of the base 31 and the cover 21, and as a result of this the base 31 and the cover 21 are welded to form a single piece constituting the housing 12. In this process, ultrasonic waves are also applied to the bottom plate 41 at the same time, and therefore the corner parts 43 of the bottom plate 41 which bite into the base 31 are also oscillated and heated. When this occurs, the resin material around each of the corner parts 43 is fused by the heat, and resin fusion parts 35 are formed in six locations so as to cover each of the corner parts 43 (see Figs. 5, 6(a) and (b)). As a result, the bottom plate 41 is fixed more securely by welding to the base 31. Fig. 6(c) shows a bottom plate 100 affixed to the base 31 utilizing conventional ultrasonic welding methods. Since the bottom plate 100 in the conventional example does not have any particular corner parts, the outer peripheral edge 42 of the bottom plate 100 does not bite into the inside surface 34a of the base 31 in the structure which is formed. In other words, the outer peripheral edge 42 of the bottom plate 100 does not extend into the inside surface 34a, so it is positioned in the center of the recess 32 rather than the inside surface 34a.

A simple description of the method of using the subcutaneous implantable port 11 according to an embodiment of the present disclosure will be given next.

As a preparatory operation for implantation of the subcutaneous implantable port 11 and the catheter 16, the skin at the site where the subcutaneous implantable port 11 and catheter 16 are to be implanted is sterilized over a wide range. A local or general anaesthetic is then given to the patient by a normal method, and the catheter 16 is implanted at the intended site using an incision technique or the Seldinger technique. The injection needle 18 is then inserted into the septum 13 and the inside of the injection liquid chamber 17 is filled with heparinized physiological saline in order to adequately purge the air. An incision is then made in the skin and a subcutaneous pocket is formed at the site where the subcutaneous implantable port 11 is to be implanted. The connection end of the catheter 16 is then cut to a suitable length, and the catheter 16 is inserted into the catheter fixing implement 15. After this, the catheter 16 is tightly inserted into the catheter connection part 14 of the subcutaneous implantable port 11, and in this state the catheter fixing implement 15 is securely attached. In this state, the injection needle 18 is used to puncture the septum 13 and heparinized physiological saline is injected in, and it is confirmed that there is a smooth flow of liquid and no liquid leakage. Finally, the subcutaneous implantable port 11 is placed inside the subcutaneous pocket and fixed by suturing the fascia; the incision wound is also sutured, and this completes the operation for the implantation of the catheter 16 and the subcutaneous implantable port 11.

Drugs etc. can be injected from about one week after the catheter 16 and subcutaneous implantable port 11 have been implanted. The skin around the edges of the site where the subcutaneous implantable port 11 is implanted is first of all thoroughly sterilized. The central part on the upper surface of the subcutaneous implantable port 11, in other words the place where the septum 13 is present, is pierced by the injection needle 18 for transcutaneous administration, and the tip end thereof is made to reach the injection liquid chamber 17 in the housing 12. In the case of this mode of embodiment, the bottom plate 41 which is made of a hard metal is positioned on the bottom part inner surface 33a of the housing 12. This means that even if the tip end of the injection needle 18 runs into the bottom plate 41, it is possible to prevent a situation in which the needle penetrates the bottom part 33. In this state, several mL of heparinized physiological saline are then injected, and the patency of the catheter 16 is confirmed, after which a drug such as an anticancer agent is injected into the blood vessel. Once the drug has been injected, several mL of heparinized physiological saline are then injected (heparin lock), and the needle is withdrawn while careful attention is paid to reverse flow to the catheter 16. As a result of the above procedure, the injection operation is complete.

The following operational advantages are demonstrated by this mode of embodiment as described above.

With the subcutaneous implantable port 11 according to this embodiment, the metal bottom plate 41 is positioned on the bottom part inner surface 33a of the base 31 which forms part of the housing 12. Consequently, it is possible to prevent the tip end of the injection needle 18 from penetrating the bottom part 33. Furthermore, the six corner parts 43 of the bottom plate 41 are made to tightly bite into the inside surface 34a of the recess 32 by press-fitting the bottom plate 41 into the recess 32 along the recess depth direction. This means that even if the resin around the bottom plate 41 melts to some extent during the ultrasonic welding step, there is no shift in the position of the bottom plate 41 or detachment thereof. A very reliable structure can therefore be produced. Furthermore, there is no need to rely on a retaining lip or separate fixing parts, as in the prior art. It is therefore possible to securely fix the bottom plate 41 to the bottom part 33 of the base 31 without complicating the procedure or increasing costs.

With the subcutaneous implantable port 11 according to this embodiment, the bottom plate 41 has a regular hexagonal shape when viewed from above and the recess 41 has a circular shape when viewed from above. Furthermore, the maximum dimension D2 of the bottom plate 41 in the planar direction is equal to or less than the inner diameter D3 of the opening 34b in the recess 32 and greater than the inner diameter D1 of the deepest part of the recess 32. This means that the bottom plate 41 can be easily conducted into the opening 34b in the recess 32 and attached by press-fitting the bottom plate 41. Furthermore, the plurality of corner parts 43 of the attached bottom plate 41 can be made to bite into the inside surface 34a of the recess 32 as the bottom plate enters the deepest part of the recess 32.

With the subcutaneous implantable port 11 according to this embodiment, the plurality of resin fusion parts 35 are present in correspondence to the places where the plurality of corner parts 43 bite into the inside surface 34a of the recess 32. The plurality of resin fusion parts 35 cover each of the plurality of corner parts 43. That is to say, the resin fusion parts 35 are not present around the whole circumference of the bottom plate 41, rather the plurality of resin fusion parts 35 are present in parts. Therefore, the resin fusion parts 35 are unlikely to become thin. This means that it is simple to achieve improved fixing strength for the bottom plate 41 because the plurality of corner parts 43 are each covered by the plurality of resin fusion parts 35.

With the subcutaneous implantable port 11 according to this embodiment, the thickness T1 of the outer peripheral edge 42 of the bottom plate 41 where the six corner parts 43 are present is less than the thickness T2 at the center of the bottom plate 41. In this case, the resistance to receiving each of the corner parts 43 is relatively small when each of the corner parts 43 is made to bite into the inside surface 34a of the recess 32. Consequently, each of the corner parts 43 can easily and reliably be made to bite in. This means that it is possible to increase the amount by which each corner part 43 bites in, so the bottom plate 41 can be fixed more securely.

With the method for producing the subcutaneous implantable port 11 according to this embodiment, as described above, when the bottom plate 41 is press-fitted into the recess 32 along the recess depth direction in the bottom plate fixing step, the plurality of corner parts 43 bite into the inside surface 34a of the recess 32. As a result, the bottom plate 41 is fixed to the bottom part 33 of the base 31. When ultrasonic waves are applied in the subsequent ultrasonic welding step, the base 31 and cover 21 are welded into a single piece, thereby forming the housing 12. Therefore, according to this production method, there is no particular increase in the number of steps or components when the bottom plate 41 is fixed. It is therefore possible to produce the abovementioned superior subcutaneous implantable port 11 in a relatively simple manner.

According to the abovementioned production method, not only is the boundary region of the base 31 and the cover 21 fused and welded when ultrasonic waves are applied in the ultrasonic welding step, the biting portions 37 of the bottom plate 41 are also oscillated to produce heat. At this point, the resin material around the biting portions 37 is fused, and the resin fusion parts 35 which cover each of the corner parts 43 of the bottom plate 41 are formed. As a result, the bottom plate 41 is more securely fixed by welding to the base 31. This means that it is possible to avoid a greater number of steps.

From the foregoing and with reference to the various figure drawings, those skilled in the art will appreciate that certain modifications can also be made to the present disclosure without departing from the scope of the same. For example, while the subcutaneous implantable port 11 is disclosed herein for injecting drugs such as anticancer agents, the subcutaneous implantable port 11 may be configured for injecting nutrients.

In the embodiment described above, a metal bottom plate was used as the bottom plate 41 made of a harder material than the base 31 etc., but a ceramic bottom plate may be used instead of this, for example.

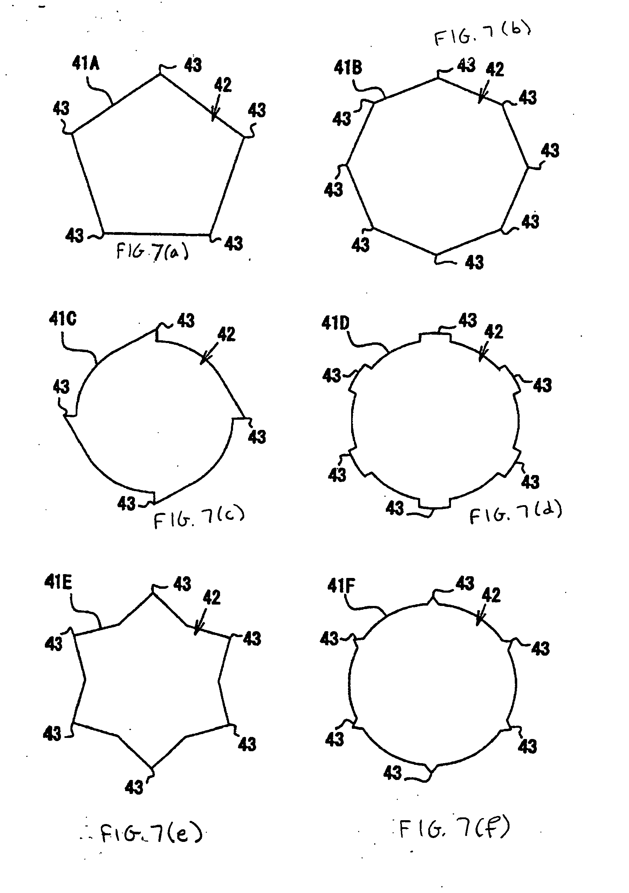

While the bottom plate 41 has been described herein as having a hexagonal shape, other shapes are contemplated. For example, it is equally possible to use a regular pentagonal bottom plate 41A having five corner parts 43, as in the different mode of embodiment shown in Fig. 7(a). Alternatively, it is equally possible to use a regular octagonal bottom plate 41B having eight corner parts 43, as in the different mode of embodiment shown in Fig. 7(b).

In the abovementioned embodiment, the regular polygonal bottom plates 41, 41A and 41B were presented as examples, but it is equally possible to employ the structures of the different modes of embodiment shown in Figs. 7(c)-7(f). The bottom plate 41C shown in Fig. 7(c) has a structure in which right-angled triangular corner parts 43 project from four points on the circular bottom plate main body. The bottom plate 41D shown in Fig. 7(d) has a structure in which substantially rectangular corner parts 43 project from six points on the circular bottom plate main body. The bottom plate 41E shown in Fig. 7(e) has a star-shaped structure comprising six corner parts 43. The bottom plate 41F shown in Fig. 7(f) has a structure in which small equilateral triangle-shaped corner parts 43 project from six points on the circular bottom plate main body. It should be noted that the shapes in the modes of embodiment described above are all rotationally symmetrical taking the center of the bottom plate as a reference.

In the embodiment described above, polypropylene resin which is relatively soft and does not chip easily was selected as the material for the base 31, but it is equally possible to select a synthetic resin other than polypropylene resin.

In the embodiment described above, the shape of the recess 32 was circular when viewed from above, but a non-circular shape is equally feasible provided that it allows the bottom plate 41 to be fixed by press-fitting.

In the embodiment described above, the examples disclosed related to cases in which the corner parts 43 had a relatively acute angle, but corner parts 43 having a slightly obtuse angle are equally feasible, provided that they are sufficiently sharp to be oscillated by the ultrasonic waves during the ultrasonic welding step.

While several embodiments of the disclosure have been shown in the drawings, it is not intended that the disclosure be limited thereto. Therefore, the above description should not be construed as limiting, but merely as exemplifications of particular embodiments. Those skilled in the art will envision other modifications within the scope of the claims appended hereto.

## Claims

1. A subcutaneous implantable port (11) comprising:
a housing (12) having a base (31) including a bottom portion (33) with a recess (32) defining an injection liquid chamber (17) and an opening of enlarged diameter, the housing having a cover (21) positioned over the base, wherein the cover defines an opening therethrough;
a septum (13) positioned between the cover (21) and the base (31) adjacent the injection liquid chamber (17), the septum (13) being configured to be penetrated by an injection needle (18) through the opening defined by the cover (21);
a bottom plate (41) disposed on an inner surface of the bottom portion (33) of the base; and
a catheter connecting portion (14) disposed on the outer surface of the housing (12),
**characterised in that** the bottom plate (41) has a plurality of corner parts (43) on an outer peripheral edge (42) thereof;
wherein the plurality of corner parts (43) bite into an inside surface of the recess (32) as the bottom plate (41) is press-fitted into the recess (32) along the recess depth direction, whereby the bottom plate (41) is fixed to the bottom part (33) of the base (31).

2. A subcutaneous implantable port (11) according to claim 1, wherein the bottom plate (41) is polygonal, and the recess (32) is circular, and the maximum dimension of the bottom plate (41) in a planar direction is equal to or less than the inner diameter of the opening in the recess (32) and greater than the inner diameter of the deepest part of the recess (32).

3. A subcutaneous implantable port (11) according to claim 2, wherein a plurality of resin fusion parts (35) are positioned adjacent to where the plurality of corner parts (43) bite into the inside surface of the recess (32), and the plurality of resin fusion parts (35) cover each of the plurality of corner parts (43).

4. A subcutaneous implantable port (11) according to claim 3, wherein the thickness of the outer peripheral edges (42) of the bottom plate (41) where the plurality of corner parts (43) are present is less than the thickness of the central part of the bottom plate (41).

5. A method for producing a subcutaneous implantable port (11), the method including the step of:
fixing a plate (41) to a bottom part (33) of a base (31) of the subcutaneous implantable port (11) by press-fitting the plate (41) into a recess of the bottom portion (33) along a recess (32) depth in a direction under unheated conditions, **characterised in that** the plate (41) includes a plurality of corner parts (43) configured to bite into an inside surface of the recess (32) so that the plate (41) is fixed to the bottom part of the base (31); and the method including the further step of ultrasonic welding the base (31) to a cover (21) to fuse the base (31) to the cover (21).

6. A method for producing a subcutaneous implantable port (11) according to claim 5, wherein, in the ultrasonic welding step, ultrasonic waves are also applied to the places where the plurality of corner parts (43) bite into the inside surface of the recess (32) in order to form a plurality of resin fusion parts (35), whereby the bottom plate (41) is simultaneously fixed by fusion to the base (31).

## Patentansprüche

1. Subkutan implantierbarer Port (11), umfassend:
ein Gehäuse (12) mit einer Basis (31) mit einem Bodenabschnitt (33) mit einer Vertiefung (32), die eine Injektionsflüssigkeitskammer (17) definiert, und eine Öffnung mit vergrößertem Durchmesser, wobei das Gehäuse eine über der Basis angeordnete Abdeckung (21) aufweist, worin die Abdeckung eine Öffnung durch sie hindurch definiert;
ein zwischen der Abdeckung (21)und der Basis (31) neben der Injektionsflüssigkeitskammer (17) positioniertes Septum (13), das zur Penetration durch eine Injektionsnadel (18) durch die von der Abdeckung (21) definierte Öffnung ausgelegt ist;
eine auf einer Innenseite des Bodenabschnitts (33) der Basis angeordnete Bodenplatte (41); und
einen auf der Außenseite des Gehäuses (12) angeordneten Katheterverbindungsabschnitt (14),
**dadurch gekennzeichnet, dass** die Bodenplatte (41) eine Vielzahl von Eckteilen (43) auf einem Umfangsrand (42) aufweist;
worin die Vielzahl von Eckteilen (43) in eine Innenseite der Vertiefung (32) greifen, wenn die Bodenplatte (41) in die Vertiefung (32) entlang der Vertiefungstiefenrichtung eingedrückt wird, wodurch die Bodenplatte (41) am Bodenteil (33) der Basis (31) fixiert wird.

2. Subkutaner implantierbarer Port (11) nach Anspruch 1, worin die Bodenplatte (41) polygonal ist und die Vertiefung (32) kreisförmig ist und die maximale Abmessung der Bodenplatte (41) in einer ebenflächigen Richtung gleich oder kleiner als der Innendurchmesser der Öffnung in der Vertiefung (32) und größer als der Innendurchmesser des tiefsten Teils der Vertiefung (32) ist.

3. Subkutaner implantierbarer Port (11) nach Anspruch 2, worin eine Vielzahl von Harzfusionsteilen (35) neben der Stelle angeordnet sind, an der die Vielzahl von Eckteilen (43) in die Innenseite der Vertiefung (32) greifen und die Vielzahl von Harzfusionsteilen (35) jeden der Vielzahl von Eckteilen (43) abdecken.

4. Subkutaner implantierbarer Port (11) nach Anspruch 3, worin die Dicke der äußeren Umfangsränder (42) der Bodenplatte (41), wo die Vielzahl von Eckteilen (43) vorliegen, kleiner als die Dicke des Mittelteils der Bodenplatte (41) ist.

5. Verfahren zur Herstellung eines subkutanen implantierbaren Ports (11), das folgenden Schritt umfasst:
Fixierung einer Platte (41) an einem Bodenteil (33) einer Basis (31) des subkutanen implantierbaren Ports (11) durch Eindrücken der Platte (41) in eine Vertiefung des Bodenteils (33) entlang einer Vertiefungstiefe (32) in einer Richtung unter ungeheizten Bedingungen, **dadurch gekennzeichnet, dass** die Platte (41) eine Vielzahl von Eckteilen (43) aufweist, die zum Eingreifen in eine Innenseite der Vertiefung (32) ausgelegt sind, so dass die Platte (41) am Bodenteil der Basis (31) fixiert wird; und worin das Verfahren den weiteren Schritt des Ultraschallschweißens der Basis (31) an einer Abdeckung (21) zum Anschmelzen der Basis (31) an der Abdeckung (21) aufweist.

6. Verfahren zur Herstellung eines subkutanen implantierbaren Ports (11) nach Anspruch 5, worin im Ultraschallschweißschritt Ultraschallwellen auch auf die Stellen aufgebracht werden, an denen die Vielzahl von Eckteilen (43) in die Innenseite der Vertiefung (32) greifen, um eine Vielzahl von Harzfusionsteilen (35) zu formen, wodurch die Bodenplatte (41) gleichzeitig durch Fusion an der Basis (31) fixiert wird.

## Revendications

1. Orifice implantable sous-cutané (11) comprenant :
un boîtier (12) ayant une base (31) qui comporte une partie inférieure (33) avec un évidement (32) définissant une chambre de liquide d'injection (17) et une ouverture de diamètre élargi, le boîtier ayant un couvercle (21) positionné sur la base, où le couvercle définit une ouverture à travers celui-ci ;
un septum (13) positionné entre le couvercle (21) et la base (31) de manière adjacente à la chambre de liquide d'injection (17), le septum (13) étant configuré pour être pénétré par une aiguille d'injection (18) à travers l'ouverture définie par le couvercle (21) ;
une plaque inférieure (41) disposée sur une surface interne de la partie inférieure (33) de la base ; et
une partie de raccordement de cathéter (14) disposée sur la surface externe du boîtier (12),
**caractérisé en ce que** la plaque inférieure (41) comporte une pluralité de parties de coin (43) sur son bord périphérique externe (42) ;
où la pluralité de parties de coin (43) mordent dans une surface intérieure de l'évidement (32) à mesure que la plaque inférieure (41) est ajustée par pression dans l'évidement (32) le long de la direction de profondeur de l'évidement, moyennant quoi la plaque inférieure (41) est fixée à la partie inférieure (33) de la base (31).

2. Orifice implantable sous-cutané (11) selon la revendication 1, dans lequel la plaque inférieure (41) est polygonale, et l'évidement (32) est circulaire, et la dimension maximale de la plaque inférieure (41) dans une direction plane est inférieure ou égale au diamètre interne de l'ouverture dans l'évidement (32) et supérieure au diamètre interne de la partie la plus profonde de l'évidement (32).

3. Orifice implantable sous-cutané (11) selon la revendication 2, dans lequel une pluralité de parties de fusion de résine (35) sont positionnées de manière adjacente à l'endroit où la pluralité de parties de coin (43) mordent dans la surface intérieure de l'évidement (32), et la pluralité de parties de fusion de résine (35) couvrent chacune de la pluralité de parties de coin (43).

4. Orifice implantable sous-cutané (11) selon la revendication 3, dans lequel l'épaisseur des bords périphériques externes (42) de la plaque inférieure (41), où la pluralité de parties de coin (43) sont présentes, est inférieure à l'épaisseur de la partie centrale de la plaque inférieure (41).

5. Procédé de production d'un orifice implantable sous-cutané (11), le procédé comportant l'étape qui consiste :
à fixer une plaque (41) à une partie inférieure (33) d'une base (31) de l'orifice implantable sous-cutané (11) en ajustant par pression la plaque (41) dans un évidement de la partie inférieure (33) le long d'une profondeur d'évidement (32) dans une direction sous des conditions non chauffées, **caractérisé en ce que** la plaque (41) comporte une pluralité de parties de coin (43) configurées pour mordre dans une surface intérieure de l'évidement (32) de sorte que la plaque (41) soit fixée à la partie inférieure de la base (31) ; et le procédé comportant l'étape supplémentaire qui consiste à souder par ultrasons la base (31) à un couvercle (21) afin de fusionner la base (31) au couvercle (21) .

6. Procédé de production d'un orifice implantable sous-cutané (11) selon la revendication 5, dans lequel, dans l'étape de soudage par ultrasons, des ondes ultrasonores sont également appliquées aux endroits où la pluralité de parties de coin (43) mordent dans la surface intérieure de l'évidement (32) afin de former une pluralité de parties de fusion de résine (35), moyennant quoi la plaque inférieure (41) est simultanément fixée par fusion à la base (31).
